# NOUVEAU FASCICULE DE BREVET EUROPEEN

(11) **EP 0 122 209 B2**
(45) Date de publication et mention de la décision concernant l'opposition: **30.11.1994**
(45) Mention de la délivrance du brevet: 17.01.1990
(21) Numéro de dépôt: 84430010.3
(22) Date de dépôt: 03.04.1984
(51) Int. Cl.: A61K 39/44

(54) **Procédé de fixation de macromolécules biologiques sur des supports**
Verfahren zur Bindung von biologischen Makromolekülen auf Trägern
Process for binding biological macromolecules to carriers

(30) Priorité: 06.04.1983 FR 8305618
(43) Date de publication de la demande: 17.10.1984
(73) Titulaire: Société Anonyme dite: IMMUNOTECH, F-13288 Marseille Cedex 9 (FR)
(72) Inventeur: Delaage, Michel, F-13001 Marseille (FR); Drocourt, Jean-Louis, c/o Résidence de l'Ailhaude, F-13008 Marseille (FR); Prince, Paul, F-13009 Marseille (FR)
(74) Mandataire: Rinuy, Santarelli

(56) Documents cités:
- EP-A- 0 071 976
- DE-A- 2 840 503
- FR-A- 1 604 982
- FR-A- 2 028 702
- FR-A- 2 035 774
- US-A- 4 069 352
- US-A- 4 282 287
- M. ROITT: "Essential immunology", édition 4, pages 144-151, Blackwell Scientific Publications, 1980, Oxford, GB;
- Chem.Abstracts vol.91,ref. 209046t
- Journal of Histochemistry and Cytochemistry, vol. 27 pp 1131-1139(1979)
- WISEMAN, A. Topics in Enzyme and Fermentation Biotechnology, 1978. .
- Römmps Chemie Lexicon, Franck'sche. Verlagsbuchandlung Stuttgart 1987, Seite 3288.

## Description

La présente invention concerne un procédé de fixation de macromolécules biologiques sur des supports.

On sait que l'utilisation de macromolécules biologiques immobilisées sur un support solide répond à de nombreux besoins dans le domaine de la recherche médicale ou biologique, de la biotechnologie ou du génie bio-médical.

Les macromolécules concernées sont, par exemple, du type des enzymes tels que ceux utilisés pour réaliser des bio-réacteurs dans lesquels on traite les solutions à transformer. Ce sont aussi, par exemple, des anticorps fixés sur des tubes ou sur des billes que l'on utilise pour les dosages radioimmunologiques ou enzymoimmuno- logiques.

De même, un grand nombre de macromolécules biologiques sont utilisées en phase fixe pour la chromatographie de leurs ligands naturels: hormones pour purifier le récepteur, substrat pour purifier l'enzyme, antigène pour un anticorps ou vice-versa.

Les procédés utilisés jusqu'ici pour fixer de telles macromolécules sont fondés sur deux concepts, à savoir essentiellement:
1. Le couplage chimique qui implique que le support et la macromolécule à fixer présentent des fonctions convenables susceptibles de réagir entre elles. C'est ainsi que s'il s'agit d'un support en un matériau polymère, on peut faire apparaître, soit au moment de la synthèse de ce polymère, soit ultérieurement, des fonctions réactives (carboxyliques par exemple) qui seront appelées à réagir avec les fonctions réactives présentes dans la macromolécule (fonctions aminées, par exemple) au moyen d'agents d'activation classiques comme les carbodiimides ou les alkylchlorocarbonates. On peut également faire apparaître des fonctions aminées sur le support, fonctions qui seront liées à des fonctions réactives déjà présentes sur la macromolécule (fonctions carboxyliques par exemple). Ainsi, les polyamides ("Nylon") traitées par une base forte s'hydrolysent partiellement en surface et laissent apparaître des fonctions carboxyliques ou aminées, fonctions qui sont susceptibles de réagir respectivement avec des fonctions aminées ou carboxyliques déjà présentes dans une macromolécule à fixer sur un support de ce type polyamide. De même, dans les séries polysaccharidiques qui sont très utilisées comme supports chromatographiques, on peut créer des groupements réactifs par l'anion pé- riodate ou par le bromure de cyanogène, sur lesquelles peuvent s'additionner les fonctions aminées d'une macromolécule. On pourrait ainsi multiplier les exemples illustrant ce premier concept fondé sur le couplage chimique comme le couplage diazo, les liaisons par semi-quinone, etc.
2. L'affinité constatée de certaines macromolécules pour tel ou tel support donné. La fixation se fait alors spontanément par liaison non cova- lente. Le cas le plus connu est celui des anticorps (immunoglobulines) qui adhérent sur des supports polymères hydrophobes, du type poly- vinyle ou polystyrène. On connaît également le cas des peptides cationiques qui adhérent spontanément sur les supports en verre.

Les procédés rappelés ci-dessus présentent des avantages et des inconvénients.

Dans le premier cas, on obtient des liaisons solides mais au prix d'opérations chimiques sur le support, opérations qui ne sont pas toujours réalisables à grande échelle et qui limitent l'éventail des supports possibles.

Dans le second cas, le domaine d'application est encore plus limité du fait qu'il dépend de l'affinité d'une macromolécule donnée pour un support donné ne permettant pas d'étendre cette propriété à tous les supports. De plus, les surfaces obtenues sont fragiles et peu stables dans le temps. En outre, dans l'un comme dans l'autre cas, la quantité de macromolécules à fixer ne peut être maîtrisée.

La présente invention vise un procédé obviant aux inconvénients des procédés antérieurs rappelés ci-dessus et permettant, d'une part; une fixation solide et durable d'une macromolécule biologique quelconque sur un support quelconque, et d'autre part, le contrôle de la quantité fixée de cette macromolécule.

Ce procédé est essentiellement caractérisé par le fait qu'il consiste à revêtir le support d'une couche d'une macromolécule biologique qui est un antigène ou un anticorps et à réticuler les macromolécules biologiques ainsi déposées à l'aide d'un agent de réticulation chimique, qui est le dichlorure de succinyle formant ainsi un revêtement ayant une affinité pour le support supérieure à celle de ladite macromolécule et renfermant une quantité contrôlée de cette dernière et en ce que le support est en matière plastique.

Suivant un mode de mise en oeuvre possible, le revêtement formé ayant une plus grande afinté pour le support résulte de la réticulation des macromolécules biologiques préalablement déposées sur ce support. La demanderesse a en effet trouvé que certaines macromolécules biologiques ayant peu d'affinité pour un support présentent une affinité supérieure pour ce dernier lorsqu'elles se trouvent à l'état réticulé. on utilise selon l'invention comme agent de réticulation chimique, le dichlorure de succinyle, de préférence en phase vapeur.

On peut illustrer ces modes de mise en oeuvre de la façon suivante: en désignant par A la macromolécule biologique connue pour avoir une faible affinité pour un support donné ou peur être difficile à fixer sur ce support
- Selon un mode de réalisation, on réticule cette macromolécule A avec elle-même. Le revêtement réalisé sur le support présente alors une adhésivité très supérieure à celle du monomère;
- Selon un autre mode de réalisation, on couple ou associe à la macromolécule A une macromolécule B et, après une opération de polymérisation ou de réticulation, on obtient un ensemble polymère donnant un revêtement hautement adhésif;
- Selon encore un autre mode de réalisation, on réalise sur le support un revêtement au moyen de B (polymérisé ou non) destiné à recevoir et à fixer A, après couplage, réticulation ou polymérisation; l'ensemble forme alors un revêtement final hautement adhérent.

En procédant conformément à l'invention, il est ainsi possible de contrôler ou de régler la quantité de la macromolécule biologique à fixer sur un support comme on le verra ci-après.

Elle est applicable à tout support en matériaux plastiques tels que polystyrènes, polyéthylènes, polyvinyles et analogues, sans limitation de formes ou de dimensions.

Les exemples suivants sont donnés à titre illustratif et nullement limitatif de l'invention.

### Exemple 1:

### Fixation directe d'un anticorps sur un tube de polys tyrène:

On sait que les anticorps de lapin et d'autres espèces animales adhérent de façon plus ou moins stable au polystyrène et autres matières plastiques (polyvinyles, polycarbonate, etc). Ce phénomène est à la base de nombreuses applications notamment dans le domaine de l'immunoanalyse (dosages radioimmunologiques ou immunoenzymati- ques).

Cet exemple illustre la stabilisation de cette fixation conformément à l'invention.

Un tube de polystyrène (tube à hémolyse 13 x 75 mm) reçoit une solution d'anticorps à 0,1 mg/ml dans un tampon neutre ou légèrement alcalin. Après 4 heures d'incubation à température ambiante, la surface du tube a adsorbé environ 1 ug d'anticorps par cm².

Pour stabiliser la fixation et selon l'invention:
- On sèche le tube;
- On réalise la polymérisation de l'anticorps en exposant le tube sec à une tension de vapeur saturante de dichlorure de succinyle;
- On rince à l'eau et sèche.

On constate que la fixation est stabilisée: l'anticorps ne peut plus être enlevé même en présence d'un détergent comme le "TRITON@ X 100".

### Exemple 2.

### Fixation d'héparine sur les biomatériaux

La fixation d'héparine vise à résoudre un vieux problème, celui de la compatibilité des matériaux au contact du sang, soit dans le cas de prothèse, soit dans une utilisation extracorporelle (dialyse rénale par exemple). Les propritétés mouillantes et anticoagulantes de l'héparine empêchent la coagulation sanguine au contact du matériau. La fixation de l'héparine peut être réalisée dans les conditions suivantes:
- On prépare une solution contenant 1 g/I d'albumine et 1 g/I d'héparine dans un tampon morpholinoéthane sulfonique 50 mM pH 5,5. La polymérisation est effectuée au moyen de 1 g/I de 1 éthyl 3-3 diméthylaminopropylcar- bodiimde. Après 1 heure de polymérisation, la solution est diluée 10 fois.

Il suffit alors d'immerger le matériau pendant 4 heures pour obtenir une fixation efficace du copolymère albuminehéparine. Dans le cas de matériaux insuffisamment hydrophobes, ou sur le verre, on peut compléter le traitement, après séchage, par une exposition en vapeur de dichlorure de succinyle, comme décrit dans l'exemple 1. Le relais avidi- ne-biotine peut être aussi utilisé

Tubulures, filtres, modules de dialyse rénale, implants, verres de contact, peuvent être traités de la sorte.

On notera aussi tout particulièrement que le procédé selon l'invention permet d'obtenir le revêtement par des macromolécules biologiques de billes (notamment de polyéthylène) présentant une faible densité (inférieure à celle du solvant ou du milieu dans lequel elles sont utilisées), conduisant ainsi à des produits dont on ne peut disposer à l'heure actuelle et dont on comprendra tout l'intérêt. Ces billes constituent donc des produits industriels nouveaux.

## Revendications

1. Procédé de fixation solide et durable d'une macromolécule biologique sur un support solide chimiquement neutre pour lequel elle a une faible affinité, procédé caractérisé en ce qu'il consiste à revêtir le support d'une couche de ladite macromolécule biologique qui est un antigène ou un anticorps, et a réticuler les macromolécules biologiques ainsi déposées à l'aide d'un agent de réticulation chimique qui est le dichlorure de succinyle, formant ainsi un revêtement ayant une affinité pour le support supérieure a celle de ladite macromolécule et renfermant une quantité contrôlée de cette dernière, et en ce que le support est en matière plastique.

2. Procédé de fixation selon la revendication 1, caractérisé en ce que ledit dichlorure de succinyle est appliqué en phase vapeur.

## Claims

1. A process for the solid and durable binding of a biological macromolecule to a chemically inert solid carrier for which it has a low affinity, the process being characterised in that it comprises coating the carrier with a layer of said biological macromolecule which is an antigen or an antibody and of polymerising the biological macromolecules thus deposited by means of a chemical polymerising agent which is succinyl dichloride, thereby forming a coating having an affinity for the carrier higher than that of said macromolecule and containing a controlled amount of this latter and in that the carrier is a plastic material.

2. A binding process according to claim 1, characterised in that said succinyl dichloride is applied in vapour phase.

## Patentansprüche

1. Verfahren zum festen und dauerhaften Binden eines biologischen Makromoleküls an einen festen, chemisch neutralen Träger, für den es eine geringe Affinität aufweist, welches Verfahren dadurch gekennzeichnet ist, daß der Träger mit einer Schicht des biologischen Makromoleküls, welches ein Antigen oder ein Antikörper ist, überzogen wird und die so aufgebrachten biologischen Makromoleküle mit einem chemischen Vernetzungsmittel, welches Succinyldichlorid ist, vernetzt werden, wodurch ein Überzug gebildet wird, der eine höhere Affinität für den Träger als das Makromolekül aufweist und eine kontrollierte Menge desselben enthält, und daß der Träger aus Plastikmaterial ist.

2. Bindungsverfahren nach Anspruch 1, dadurch gekennzeichnet, daß das Succinyldichlorid in Dampfphase aufgebracht wird.
